## Europäisches Patentamt
## European Patent Office
## Office européen des brevets

(19)

(11) Publication number: **0 261 809 B1**

(12)
# EUROPEAN PATENT SPECIFICATION

(45) Date of publication of patent specification: **16.10.91**

(51) Int. Cl.⁵: **A61F 5/02, A61F 5/34**

(21) Application number: **87307552.7**

(22) Date of filing: **26.08.87**

(54) Therapeutic lumbosacral appliance.

(30) Priority: **29.08.86 US 901559**

(43) Date of publication of application:
**30.03.88 Bulletin 88/13**

(45) Publication of the grant of the patent:
**16.10.91 Bulletin 91/42**

(84) Designated Contracting States:
**AT BE CH DE FR GB LI LU NL SE**

(56) References cited:
**US-A- 4 135 503**
**US-A- 4 178 922**
**US-A- 4 178 923**

(73) Proprietor: **Safeguard Industrial Corporation
Leisz Road
Leesport Pennsylvania 19533(US)**

(72) Inventor: **Sebastian, Peter Raymond
919 Loch Lomond Court
Salisbury MD 21801(US)**
Inventor: **Stump, John L.
Grande Pointe No. 1202 133 Lakefront Drive
Daphne AL 36526(US)**
Inventor: **Sebastian, Thomas Vincent
1410 Rose Virginia Road
Reading PA 19611(US)**

(74) Representative: **Brooke-Smith, Fred et al
Stevens, Hewlett & Perkins 1 Serjeants' Inn
Fleet Street
London EC4Y 1LL(GB)**

EP 0 261 809 B1

## Description

The present invention relates to a lumbosacral therapeutic appliance designed to relieve pain in the areas of the lumbosacral spine and sacroiliac joints by maintaining normal alignment of the osseous structures and providing static stretch to paravertebral muscles to alleviate unwanted muscle spasms and fatigue.

The problem of low back pain is commonly a result of mechanical and physiologic derangements of the osseous ligamentous and muscular structures of this region. The low back for purposes of this discussion refers to the area of the lumbar and sacral portions of the spinal column and the sacroiliac joints. The interrelationships between the osseous, ligamentous and muscular structures is highly important in this area of the spine which is responsible for considerable weight bearing and structural support.

Injury to one or more of these three elements commonly results in dysfunction and subsequent pain in the others. Also, injury at one level of the spine may affect adjacent segments leading to dysfunction distant to the original abnormality or site of injury. Proper posture for optimal function is the result of correct vertebral alignment, balanced ligamentous support and limitation of excessive or unwanted movement, and flexibly balanced, strong musculature regulating and stabilizing motion.

While these objectives may be met by a variety of therapeutic modalities, low back braces used for this purpose should have certain characteristics, such as 1) contour fitting of the normal spinal/pelvic curvatures; 2) sufficient firmness to prevent buckling or unwanted binding of the appliance during wearer movement; 3) adequate flexibility to allow free unrestricted normal range of motion; 4) adequate coverage of anatomically and functionally related segments of the spine where therapeutic forces are needed for maximal effectiveness; and 5) contour shaping of the pelvic segment to avoid unwanted bony bridging effect of the iliac crests which inhibits desired counter-pressure over midline and adjacent paravertebral muscles and osseous elements. The prior art therapeutic air inflated appliances fail in one or more of these areas of therapeutic principles.

US-A-4178923 discloses an appliance having the features described in the pre-characterizing part of Claim 1. According to the present invention such an appliance is characterized in that the lower ends of said air chambers define an arc following the upper contour of the iliac crests on each side of a central portion of said air bladder, and in that there is provided at least one anchoring air chamber disposed between said transversely extending air chambers and the lower edge of said air bladder, said anchoring air chamber or chambers extending in arcuate fashion from said central portion of said air bladder toward opposite ends of said shell and positioned to lie below the posterior superior iliac spines to prevent upward riding of said therapeutic appliance when inflated and in place on the human body and to provide support to the sacroiliac joints.

The present invention provides a new and improved lumbosacral therapeutic appliance designed to relieve pain in the areas of the lumbosacral spine and sacroilic joints by maintaining normal alignment of the osseous structures and providing static stretch to paravertebral muscles to alleviate unwanted muscle spasms and fatigue. The device of the present invention can perform this function without limitation of the normal motion of the wearer. In addition, this device provides full coverage over the above mentioned structures by being form fitted to the contours of the area while avoiding unwanted bony bridging.

Details of the invention are shown by the following description of several embodiments with reference to the figures in the drawings, in which are shown:

Fig 1 illustrates the lower part of the throacic spine, the lumbar spine, the sacrum, the sacroiliac joints, the iliac crests and the posterior superior iliac spine.

Fig 2 illustrates a cut-away view of the device according to the present invention showing the relationship of the air bladder to the lumbosacral spine and sacroiliac joints.

Fig. 3 shows a side view of the spine and the positions of the air chambers of the device according to the present invention.

Fig. 4a shows a perspective view of the device according to the present invention with an external shell enclosing the air bladder.

Fig. 4b shows a perspective view of another embodiment of the device according to the present invention with the external shell being separated into two elongated sections on either side of the air bladder.

Fig. 5a is a perspective view of the device according to the present invention which shows the outlet for an air conduit located near the front closure.

Fig. 5b is a perspective view of the device shown in Fig. 4b which shows the outlet for an air conduit located near an upper front closure.

Fig. 6 is a more detailed view of Fig. 5a.

Fig. 7 is a cut-away view showing the connection of the air conduit to the air chambers.

Fig. 8 shows a second embodiment of the present invention which includes air bladder tightening means.

Fig. 9 shows a third embodiment of the present invention which includes pockets inside the shell of the therapeutic appliance for holding hot packs or cold packs to apply heating or cooling, respectively, to the sacro lumbar region of a wearer of the device according to the present invention.

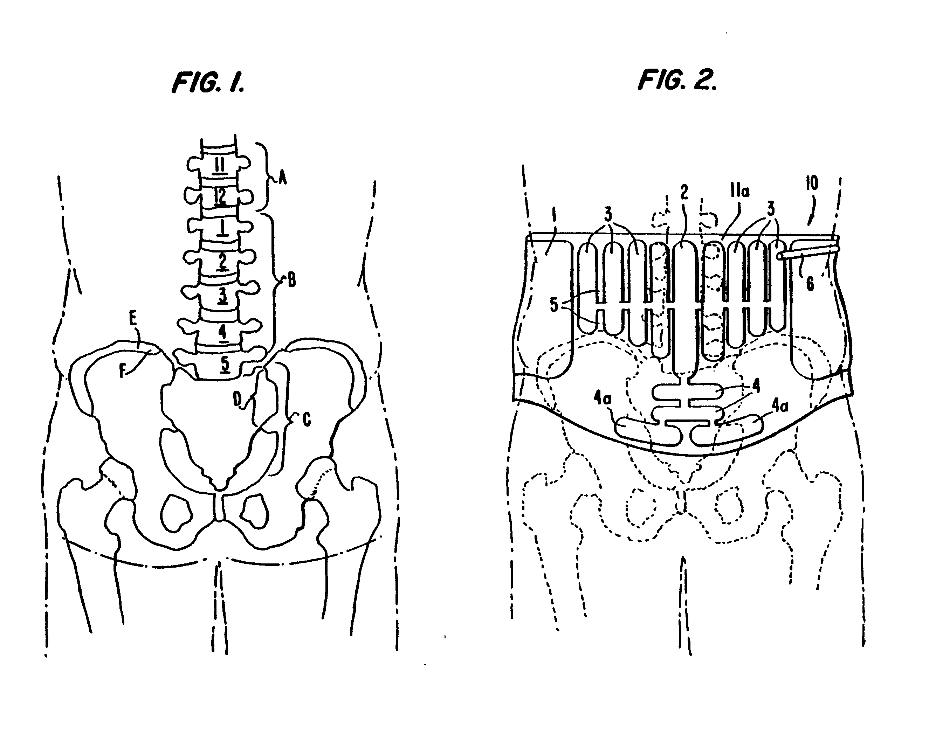

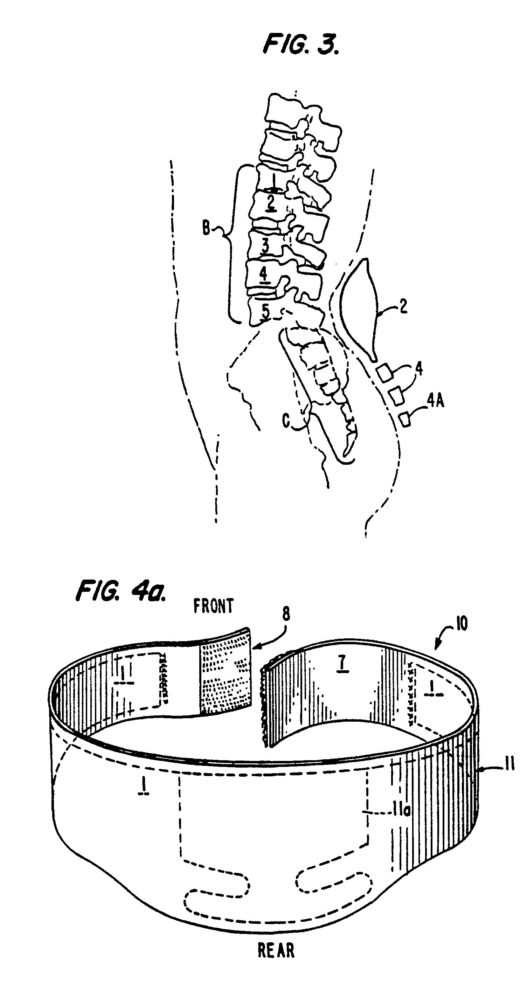

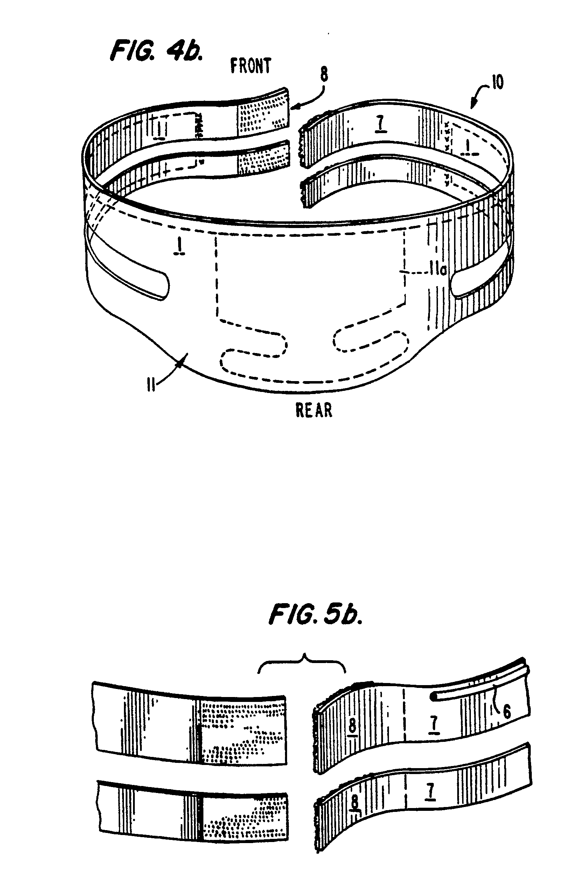

The therapeutic appliance of the present invention is generally indicated at 10 in Figures 2 and 4. Figure 1 illustrates the areas anatomically underlying the therapeutic appliance. These areas are those intended to the therapeutically affected by the appliance. The lumbar spine (B) consists of five vertebrae in normal subjects, each separated by an intervening invertebral disc. The sacrum (C) consists of five anatomically fused segments. The sacroiliac joints (D) represents the articulation of the sacrum and iliac bones on each side. The iliac crest (E) is that portion of the iliac bone lying highest and ending in the posterior superior iliac spine (F) which is the most posterior bony protuberance. Both (E) and (F) may hold prior art appliances away from the spine and paravertebral musculature to be treated thus diminishing the intended therapeutic counter-pressure.

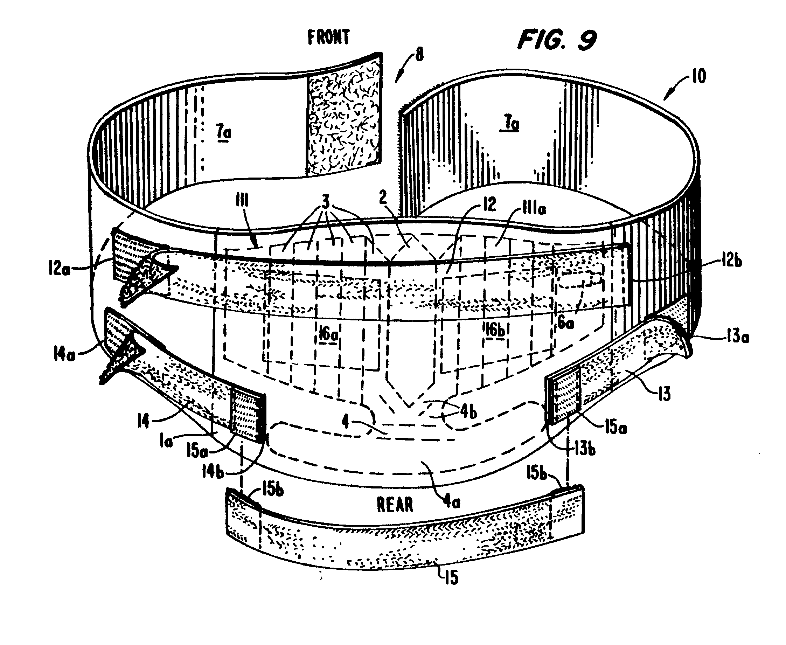

The therapeutic appliance 10 of the present invention comprises a belt which extends in a longitudinal direction and an air bladder 11 which includes a number of air chambers shown generally at 11a and a non-air filled segment 1 of the bladder, as shown in Figure 2. The air chambers preferably are 3/4 to 1 1/2 inches in width in the longitudinal direction when uninflated and comprise a central transversely extending air chamber 2 which overlies the midline of the lumbar spine and which extends vertically down to the sacrum. On either side of the central air chamber 2 are more laterally placed air chambers 3 which have lower ends which define an arc following the upper contour of the iliac crests. The upper ends of the air chambers 2 and 3 can be substantially parallel to the general lengthwise dimension of the belt as shown in Figure 2 or can lie along a path which tapers from the central air chamber 2 towards opposite ends of a modified air bladder 111, such that the lengths of the air chambers 3 become longer towards the central air chamber 2, as shown in Figure 9. Below the central air chamber 2 are at least one longitudinally extending air chamber 4 which overlies the sacrum and lies between the sacroiliac joints and arcuately extending anchoring air chambers 4a which overlie the sacroiliac joints and which lie just beneath the posterior superior iliac spines and serve additionally as anchors to prevent upward riding of the appliance and also provide support for the sacroiliac joints. The air chambers are separated by a heat sealed of sewn portion of the bladder material. It can be seen from Figures 4a and 4b that the non-air filled segment 1 of the air bladder 11 extends around a shell 7 of the therapeutic device terminating short of a closures device 8. The contoured fit of the air chambers when inflated are shaped to maintain the normal lordotic curvature of the individual wearer's spine as can be seen in Figure 3. The modified air bladder 111 shown in Figure 9 includes a central air chamber 2 having a width greater than the air chambers 3 and diagonally extending chambers 4b connect the air chambers 3 on either side of the central chamber 2 to the longitudinally extending chamber 4. The diagonally extending chambers are smaller in width than air chambers 3 and 4 to allow flexibility of the appliance in the area directly below and to either side of the central air chamber 2.

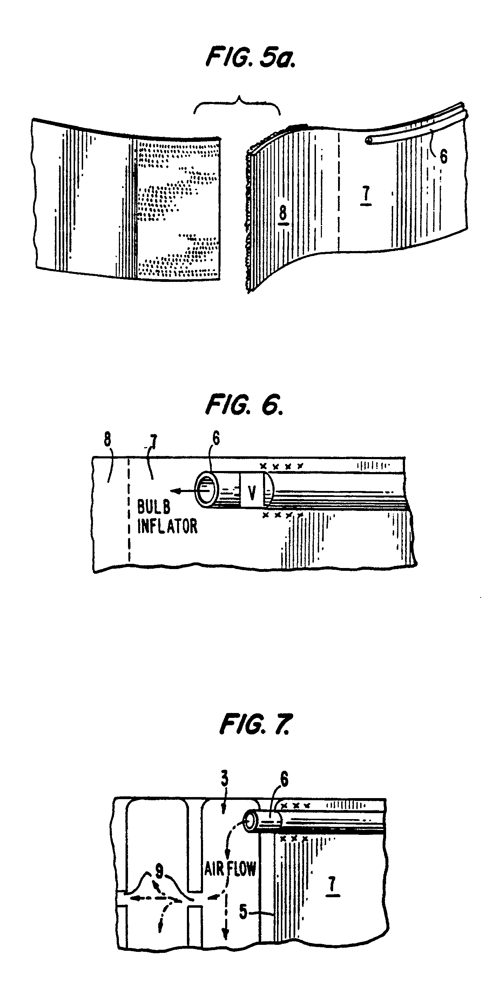

The external shell 7 of the therapeutic device of the present invention is composed of a washable moisture absorbent fabric which allows the device to be worn inside or outside of the wearer's clothing. The external shell 7 assumes the same basic shape of the contained air bladder 11 which it protects. The bladder 11 is fabricated from a nylon or plastic/vinyl material with low stretchability to allow repeated reproduction of the same air chamber configuration each time the air chambers are inflated. The therapeutic appliance can be secured over the wearer's trunk by a single closure device 8 which can be a VELCRO type of attachment, as shown in Figures 4a and 5a, or the external shell can be separated into an upper and lower section which extend longitudinally from adjacent the bladder 11 with a space therebetween and with a closure device at the free end of each section, as shown in Figures 4b and 5b. Alternatively, other fastening means such as buckles, snaps or lace-up type of attachment means can be used.

Figure 3 shows a midline sagital section of the inflated central air chambers having a tapered upper and lower end with a broad central section of the vertically aligned lumbar chamber intended to aid in the maintenance of the normal lumbar lordotic curvature. Alternatively, other configurations of the inflated air chambers are within the scope of the present invention.

Figure 5a shows the position of an air conduit 6 in the upper margin of the external shell 7 where it exits to the external surface of the shell or the air conduit can be located in the upper section of the external shell, as shown in Figure 5b. However, the air conduit 6 can be located anywhere within the bladder 11. An enlarged drawing of the outlet of the air conduit 6 is shown in Figure 6 wherein a valve (V) is shown within the air conduit for maintaining inflation at a desired level. Also, not shown is an air pump, such as a bulb inflater, which can be attached to the outlet of the air conduit 6 for inflation of the air chambers.

Fig. 7 shows the entry of the air conduit 6 into a laterally placed chamber 3. The air conduit 6 is held in position within the bladder 11 and in fluid communication with at least one of the air chambers 11a by heat sealing or sewing a segment 5 of the air bladder 11. Air passes freely from the air conduit 6 into one of the air chambers, such as a lateral chamber 3, and then through openings 9 between the air chambers for allowing the air to fill all chambers within the bladder.

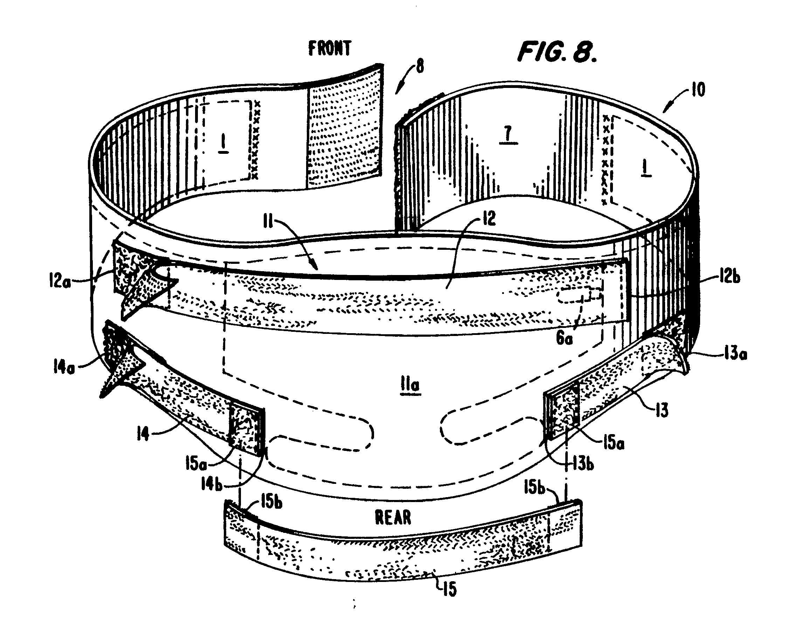

Figure 8 shows a modification of the therapeutic lumbosacral appliance wherein the external shell 7 comprises end sections 7a and an intermediate section 7b, the section 7b being of a washable moisture absorbent fabric which allows the device to be worn inside or outside of the wearer's clothing and section 7b has the same dimensions as that of the modified air bladder 111 shown in Figure 9. The modified air bladder 111 is slightly larger in length than the length in the longitudinal direction of the air chambers 11a shown in Figure 4a of the present invention. The air bladder 111 can include the configuration of air chambers as are shown in Figure 2 of the present invention or these chambers can be modified to have other configurations such as that shown in Figure 9. The section 7b of the shell 7 is attached at both longitudinal ends thereof to respective ends of the sections 7a, each of which is formed from a four-way stretch material such as neoprene or latex optionally coated on one or both sides with a nylon fabric such as a looped plush polyester. Alternatively, the sections 7a can be of a semi-rigid sheet of material such as a woven nylon webbing. The closure device 8, such as a VELCRO type of attachment, is provided at the free ends of the sections 7a.

The second preferred embodiment shown in Figures 8 and 9 also includes air bladder tightening means comprising the uninflated portion 1, 1a of the air bladder 11, 111 and any one or more of belts 12, 13, 14 and 15. The first belt 12 is attached at one end thereof to the shell 7 at a position to overlie the air chambers 2 and 3. For instance, one end 12b of the belt 12 can be attached adjacent one longitudinal end of the air chambers 11a, 111a and the other end 12a of the belt 12 can be adjustably attached by suitable means located on the section 7a of the shell 7 located adjacent the other longitudinal end of the air chambers 11a, 111a. Suitable adjustable connection means can comprise a VELCRO type attachment with the respective hook and loop sections being located on the free end 12a of the strap 12 and the section 7a of the shell 7. The function of the belt 12 is to press the upper air chambers 2, 3 tightly against the lumbar spine with the lower ends of the air chambers 3 extending in an arcuate path above the iliac crests. The belt 12 provides additional support since the length of the air chambers 2 and 3 bend the upper ends thereof tend to bend due to the curvature of the wearer's back, and without the belt 12 the upper ends of air chambers 2 and 3 are bent outwardly away from the body of the wearer when they are inflated. The belt 12 cooperates with the uninflated portion of the air bladder 1, 1a to thereby tighten the air chambers against the lumbar spine.

A pair of belts 13, 14 are provided on either side of the anchoring air chamber 4a to hold the anchoring air chamber 4a tightly against the sacroiliac joints just beneath the posterior superior iliac spines to thereby assist the anchoring air chamber 4a in preventing upward riding of the appliance and also to provide additional support for the sacroiliac joints. The belts 13, 14 can be attached at one end 13b, 14b thereof to a portion of the air bladder 1a adjacent to opposite longitudinal ends of the anchoring air chamber 4a. The free ends 13a, 14a of each of the straps 13, 14 can be attached by suitable means, such as VELCRO type hook and loop sections, to a portion of one of the sections 7a of the shell. A suitable length for the straps 13, 14 can be about 8 inches or any other suitable dimension. Likewise, a suitable length for the strap 12 can be 20 inches or any suitable dimension depending upon the particular shape of the wearer of the therapeutic lumbosacral appliance.

An optional belt 15 can be provided to overlie the anchoring air chamber 4a. Suitable means for attaching the optional belt 15 can comprise a VELCRO type attachment with the respective hook and loop sections 15a, 15b being applied to the fixed ends of the belts 13, 14 and the free ends of the optional strap 15.

In the modification shown in Figure 8, the air conduit 6 terminates in a suitable air valve 6a which is provided to extend outwardly from the air bladder 1a and through a suitably sized hole in the upper belt 12. Of course, the air valve 6a can be provided at any suitable location on the air bladder 1a or the air conduit 6 can extend around the shell 7 to another suitable location with the air valve 6a located at the end thereof.

Another modification of the present invention is shown in Figure 9 wherein inside pockets are disposed on the first section 7b of the shell 7. These pockets 16a, 16b are provided for holding suitable cooling pad for applying cooling to the sacrolumbar region or the pockets can be used for holding hot packs to apply heat to the sacrolumbar region.

## Claims

1. A therapeutic appliance for application to the lumbar spine and which follows the contours of the iliac crests and overlies the sacrum and sacroiliac joints as well as anchors below the posterior superior iliac spines of the human body, the appliance comprising an external shell (7, 7a) having a length in a longitudinal direction sufficient to extend around and encircle the abdominal region of the body, said shell having closure means (8) at opposite ends thereof for attaching said ends together, an air bladder (11, 111) disposed on said external shell (7, 7a), said air bladder having a plurality of air chambers located centrally between said opposite ends of said shell, said air chambers including elongate air chambers (2, 3) which extend transversely to said longitudinal direction, and air conduit means for inflating said air chambers with air, characterized in that the lower ends of said air chambers (2, 3) define an arc following the upper contour of the iliac crests on each side of a central portion of said air bladder, and in that there is provided at least one anchoring air chamber (4a) disposed between said transversely extending air chambers (2, 3) and the lower edge of said bladder (11), said anchoring air chamber or chambers extending in arcuate fashion from said central portion of said air bladder toward opposite ends of said shell and positioned to lie below the posterior superior iliac spines to prevent upward riding of said therapeutic appliance when inflated and in place on the human body and to provide support to the sacroiliac joints.

2. The therapeutic appliance of Claim 1, further comprising means (12, 12a, 12b) disposed on said external shell for tightening said elongate air chambers (2, 3) against the lumbar spine of a wearer of the appliance.

3. The therapeutic appliance of Claim 1 or Claim 2 wherein said air bladder (11) is a plastics material having low stretchability to allow accurate reproduction of the configuration of said air chambers, said air chambers being separated by joints between an inner and an outer layer of said air bladder.

4. The therapeutic appliance of any one the preceding claims, further comprising means (15, 15a, 15b) disposed on said external shell for tightening said anchoring air chamber or chambers (4a) against the sacroiliac joints of a wearer of the appliance.

5. The therapeutic appliance of any one the preceding claims, further comprising means (16a, 16b) disposed on said external shell (111) for holding a hot pack or cold pack against the back muscles of a wearer of the device.

6. The therapeutic appliance of any one the preceding claims wherein said shell is a washable moisture absorbent fabric metal.

7. The therapeutic appliance of any one of the preceding claims, wherein said air conduit means includes an air conduit (6) having an valve (v) at an outlet end thereof, said outlet end being adapted for connection to an air pump for filling said air chambers with air.

8. The therapeutic appliance of any one of the preceding claims, wherein said external shell (11) is divided on each side of said air bladder into an upper and lower longitudinally extending section with a space therebetween, said upper section and said lower section each having a closure means (8) at a free end thereof.

9. The therapeutic appliance of any one of the preceding claims, wherein said external shell comprises a first section on which said air bladder is disposed and two second sections, each of which is attached at one end thereof to said first section, said closure means being disposed on the free ends of said second sections.

10. The therapeutic appliance of any one of the preceding claims, wherein said air bladder incorporates at least one further air chamber (4) which is elongate in said longitudinal direction and which is disposed between said lower ends of said transversely extending air chambers (2, 3) and said anchoring air chamber or chambers (4a).

11. The therapeutic appliance of Claim 10, wherein there are two of said further air chambers, which chambers are spaced apart from each other in a direction transverse to said longitudinal direction.

12. The therapeutic appliance of Claim 10 or Claim 11, further comprising means disposed on said external shell for tightening said further air chambers against the lumbar spine of a wearer of the appliance.

**Revendications**

1. Dispositif thérapeutique destiné à être appliqué à la colonne vertébrale lombaire, qui suit les contours des crêtes iliaques en recouvrant le sacrum et les articulations sacro-iliaques et s'ancre au-dessous des épines iliaques postéro-supérieures du corps humain, lequel dispositif comporte une enveloppe extérieure (7, 7a) qui a dans une direction longitudinale une longueur suffisante pour s'étendre autour de la région abdominale du corps et entourer celle-ci, ladite enveloppe possédant, au niveau de ses extrémités opposées, des moyens de fermeture (8) destinés à attacher entre elles lesdites extrémités, une poche d'air (11, 111) disposée sur ladite enveloppe extérieure (7, 7a), poche d'air qui comporte plusieurs chambres à air situées centralement entre lesdites extrémités opposées de ladite enveloppe, lesdites chambres à air comprenant des chambres à air allongées (2, 3) qui s'étendent transversalement à ladite direction longitudinale, et des moyens formant conduite d'air pour gonfler d'air lesdites chambres à air, caractérisé en ce que les extrémités inférieures desdites chambres à air (2, 3) définissent un arc qui suit le contour supérieur des crêtes iliaques de chaque côté d'une partie centrale de ladite poche d'air, et en ce qu'il est prévu au moins une chambre à air d'ancrage (4a) disposée entre lesdites chambres à air (2, 3) qui s'étendent transversalement et le bord inférieur de ladite poche (11), ladite ou lesdites chambres à air d'ancrage s'étendant d'une manière arquée depuis ladite partie centrale de ladite poche d'air en direction d'extrémités opposées de ladite enveloppe et étant positionnées pour se situer au-dessous des épines iliaques postéro-supérieures, afin d'empêcher ledit dispositif thérapeutique de remonter lorsqu'il est gonflé et mis en place sur le corps humain et de fournir un soutien aux articulations sacro-iliaques.

2. Dispositif thérapeutique selon la revendication 1, comportant également des moyens (12, 12a, 12b) disposés sur ladite enveloppe extérieure pour serrer lesdites chambres à air allongées (2, 3) contre la colonne vertébrale lombaire d'un utilisateur du dispositif.

3. Dispositif thérapeutique selon la revendication 1 ou la revendication 2, dans lequel ladite poche d'air (11) consiste en une matière plastique faiblement extensible pour permettre une reproduction exacte de la configuration desdites chambres à air, lesdites chambres à air étant séparées par des jonctions entre une couche intérieure et une couche extérieure de ladite poche d'air.

4. Dispositif thérapeutique selon l'une quelconque des revendications précédentes, comportant aussi des moyens (15, 15a, 15b) disposés sur ladite enveloppe extérieure pour serrer ladite ou lesdites chambres à air d'ancrage (4a) contre les articulations sacro-iliaques d'un utilisateur du dispositif.

5. Dispositif thérapeutique selon l'une quelconque des revendications précédentes, comportant en outre des moyens (16a, 16b) disposés sur ladite enveloppe extérieure (111) pour maintenir un enveloppement chaud ou un enveloppement froid contre les muscles dorsaux d'un utilisateur du dispositif.

6. Dispositif thérapeutique selon l'une quelconque des revendications précédentes, dans lequel ladite enveloppe consiste en une matière textile lavable qui absorbe l'humidité.

7. Dispositif thérapeutique selon l'une quelconque des revendications précédentes, dans lequel lesdits moyens formant conduite d'air comportent une conduite d'air (6) pourvu d'une valve (v) au niveau de son extrémité de sortie, ladite extrémité de sortie étant adaptée pour être raccordée à une pompe à air en vue de remplir d'air lesdites chambres à air.

8. Dispositif thérapeutique selon l'une quelconque des revendications précédentes, dans lequel ladite enveloppe extérieure (11) est, de chaque côté de ladite poche d'air, subdivisée en une section supérieure et en une section inférieure qui s'étendent longitudinalement en étant séparées l'une de l'autre par un espace, ladite section supérieure et ladite section inférieure possédant chacune, au niveau de leur extrémité libre, des moyens de fermeture (8).

9. Dispositif thérapeutique selon l'une quelconque des revendications précédentes, dans lequel ladite enveloppe extérieure comporte une première section sur laquelle est disposée ladite poche d'air, et deux secondes sections dont chacune est, au niveau de l'une de ses extrémités, reliée a ladite première section, lesdits moyens de fermeture étant disposés sur les extrémités libres desdites secondes sections.

10. Dispositif thérapeutique selon l'une quelconque des revendications précédentes, dans lequel ladite poche d'air comprend au moins une chambre à air supplémentaire (4) allongée dans ladite direction longitudinale et disposée entre lesdites extrémités inférieures desdites chambres à air (2, 3) qui s'étendent transversalement et ladite ou lesdites chambres à air d'ancrage (4a).

11. Dispositif thérapeutique selon la revendication 10, dans lequel il est prévu deux chambres à air supplémentaires qui sont espacées l'une de l'autre dans une direction transversale à ladite direction longitudinale.

12. Dispositif thérapeutique selon la revendication 10 ou la revendication 11, comportant également des moyens disposés sur ladite enveloppe extérieure pour serrer lesdites chambres à air supplémentaires contre la colonne vertébrale lombaire d'un utilisateur du dispositif.

**Patentansprüche**

1. Therapeutische Vorrichtung zur Anwendung auf die Lendenwirbelsäule, welche Vorrichtung sich an die Konturen der Darmbeinkämme anschmiegt und über dem Sakrum und dem Sakroiliakalgelenk darüberliegt sowie unter den hinteren oberen Darmbeinstacheln des menschlichen Körpers verankert ist, wobei die Vorrichtung einen äußeren Mantel (7, 7a) umfaßt, dessen Erstreckung in eine Längsrichtung ausreicht, um sich rund um die Bauchgegend des Körpers zu erstrecken und diese zu umgeben, wobei der Mantel an seinen gegenüberliegenden Enden Schließmittel (8) aufweist, um die Enden miteinander zu verbinden, einen Luftbalg (11, 111), der auf dem äußeren Mantel (7, 7a) angeordnet ist, wobei der Luftbalg eine Vielzahl von Luftkammern aufweist, die in der Mitte zwischen den gegenüberliegenden Enden des Mantels liegen, wobei die Luftkammern längliche Luftkammern (2, 3), die sich quer zu der Längsrichtung erstrecken, und Luftleitungsmittel zum Befüllen der Luftkammern mit Luft umfassen, dadurch gekennzeichnet, daß die unteren Enden der Luftkammern (2, 3) einen Bogen beschreiben, welcher sich auf jeder Seite des Mittelteils des Luftbalgs der oberen Kontur der Darmbeinkämme folgt, und daß zumindest eine Verankerungsluftkammer (4a) vorgesehen ist, die zwischen den sich quer erstreckenden Luftkammern (2, 3) und der unteren Kante des Balgs (11) angeordnet ist, wobei sich die Verankerungsluftkammer oder -kammern bogenförmig vom Mittelteil des Luftbalgs zu den gegenüberliegenden Enden des Mantels erstrecken und so plaziert werden, daß sie unterhalb der hinteren oberen Darmbeinstacheln liegen, um zu verhindern, daß die therapeutische Vorrichtung nach oben gleitet, wenn sie aufgeblasen und am menschlichen Körper angebracht ist, und um die Sakroiliakalgelenke zu stützen.

2. Therapeutische Vorrichtung nach Anspruch 1, welche weiters Mittel (12, 12a, 12b) umfaßt, die auf dem äußeren Mantel angeordnet sind, um die länglichen Luftkammern (2, 3) um die Lendenwirbelsäule eines Trägers der Vorrichtung festziehen zu können.

3. Therapeutische Vorrichtung nach Anspruch 1 oder 2, wobei der Luftbalg (11) ein Kunststoff von geringer Dehnbarkeit ist, um eine exakte Wiedergabe der Konfiguration der Luftkammern zu ermöglichen, wobei die Luftkammern durch Verbindungen zwischen einer inneren und einer äußeren Schicht des Luftbalgs getrennt sind.

4. Therapeutische Vorrichtung nach einem der vorhergehenden Ansprüche, welche weiters Mittel (15, 15a, 15b) umfaßt, die auf dem äußeren Mantel angeordnet sind, um die Verankerungsluftkammer oder -kammern (4a) um die Sakroiliakalgelenke eines Trägers der Vorrichtung festziehen zu können.

5. Therapeutische Vorrichtung nach einem der vorhergehenden Ansprüche, welche weiters Mittel (16a, 16b) umfaßt, die auf dem äußeren Mantel (111) angeordnet sind, um eine heiße oder eine kalte Packung gegen die Rückenmuskulatur eines Trägers der Vorrichtung zu halten.

6. Therapeutische Vorrichtung nach einem der vorhergehenden Ansprüche, wobei der Mantel ein waschbares, feuchtigkeitssaugendes Gewebemetall ist.

7. Therapeutische Vorrichtung nach einem der vorhergehenden Ansprüche, wobei die Luftleitungsmittel eine Luftleitung (6) umfassen, die an einem Auslaßende ein Ventil (v) aufweist, wobei das Auslaßende für den Anschluß an eine Luftpumpe adaptiert ist, um die Luftkammern mit Luft befüllen zu können.

8. Therapeutische Vorrichtung nach einem der vorhergehenden Ansprüche, wobei der äußere Mantel (11) auf jeder Seite des Luftbalgs in einen oberen und einen unteren sich in Längsrichtung erstreckenden Abschnitt mit dazwischenliegendem Zwischenraum geteilt ist, wobei der obere Abschnitt und der untere Abschnitt an einem freien Ende ein Schließmittel (8) aufweisen.

9. Therapeutische Vorrichtung nach einem der vorhergehenden Ansprüche, wobei der äußere Mantel einen ersten Abschnitt umfaßt, auf dem der Luftbalg angeordnet ist, und zwei zweite Abschnitte, von denen jeder an einem seiner Enden am ersten Abschnitt angebracht ist, wobei die Schließmittel an den freien Enden der zweiten Abschnitte angeordnet sind.

10. Therapeutische Vorrichtung nach einem der vorhergehenden Ansprüche, wobei der Luftbalg zumindest eine weitere Luftkammer (4) umfaßt, die sich länglich in Längsrichtung erstreckt und die zwischen den unteren Enden der sich quer erstrekkenden Luftkammern (2, 3) und der Verankerungsluftkammer oder - kammern (4a) angeordnet ist.

11. Therapeutische Vorrichtung nach Anspruch 10, welche zwei der weiteren Luftkammern aufweist, die in einem Abstand voneinander quer zur Längsrichtung angeordnet sind.

12. Therapeutische Vorrichtung nach Anspruch 10 oder 11, welche weiters Mittel umfaßt, die auf dem äußeren Mantel angeordnet sind, um die weiteren Luftkammern gegen die Lendenwirbelsäule eines Trägers der Vorrichtung festziehen zu können.

FIG. 2.

FIG. I.

# FIG. 3.

# FIG. 4a.

FRONT

REAR

**FIG. 4b.**

FRONT

REAR

**FIG. 5b.**

## FIG. 5a.

## FIG. 6.

BULB
INFLATOR

## FIG. 7.

AIR FLOW

FIG. 8.

FRONT

REAR

EP 0 261 809 B1

FRONT

*FIG. 9*

REAR